(19) Europäisches Patentamt
European Patent Office
Office européen des brevets

(11) **EP 3 121 747 A1**

(12) **EUROPEAN PATENT APPLICATION**

(43) Date of publication:
**25.01.2017 Bulletin 2017/04**

(51) Int Cl.:
*G06F 19/00* (2011.01)  *G06K 9/00* (2006.01)
*G06T 7/00* (2017.01)

(21) Application number: **16175643.2**

(22) Date of filing: **22.06.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.07.2015  JP 2015144688**

(71) Applicant: **Konica Minolta, Inc.**
**Tokyo 100-7015 (JP)**

(72) Inventors:
• **Miyake, Nobuyuki**
**Tokyo, 100-7015 (JP)**
• **Muraoka, Shintaro**
**Tokyo, 100-7015 (JP)**
• **Haraguchi, Tsuyoshi**
**Tokyo, 100-7015 (JP)**
• **Hosoki, Tetsu**
**Tokyo, 100-7015 (JP)**

(74) Representative: **Henkel, Breuer & Partner**
**Patentanwälte**
**Maximiliansplatz 21**
**80333 München (DE)**

(54) **CONSOLE AND DYNAMIC IMAGE TAKING/DIAGNOSTIC SYSTEM**

(57)     To provide a console (2, 3) capable of automatically finding the important spot in a dynamic image.

The console includes a display means (24, 34) which plays back a dynamic image formed of a plurality of frame images to display according to operation of an operator; and a learning means (21, 31) which, when detecting that operation of possibly expressing interest is performed by the operator during playback of the dynamic image, performs statistical processing on a pattern of change in time dv(i)/dt of a value v(i) of an index regarding a dynamic state of a site to be examined and/or change in time of the value v(i) of the index in a frame image displayed on the display means (24, 34) at that time to learn appearance frequency of the value v(i) of the index and/or the pattern of the change in time dv(i)/dt of the value v(i) of the index.

*FIG. 10B*

**Description**

Background of the Invention

Field of the Invention

The present invention relates to a console and a dynamic image taking/diagnostic system.

Description of the Related Art

It is tried to take a dynamic image of a site to be examined by using a semiconductor image sensor such as a FPD (flat panel detector) and apply the same to diagnosis in contrast to conventional radiation still image taking and diagnosis using a film/screen and a stimulable phosphor plate (refer to JP 2010-268979 A).

Specifically, by utilizing high-speed responsiveness when reading/deleting image data of the semiconductor image sensor, pulse-like radiation is continuously emitted from a radiation source so as to match reading/deleting timing of the semiconductor image sensor to image a plurality of times in one second to image a dynamic state of the site to be examined. By sequentially displaying a series of plurality of images obtained by imaging, a doctor may recognize a series of motion of the site to be examined.

In an example of an ultrasonic diagnostic device, a magnetic resonance imaging device and the like, in JP 2002-095640 A, an imaging technician determines importance while watching a moving image displayed simultaneously with imaging and associates a marker (bookmark) with image data of the moving image such that the number is larger as the importance is higher, for example, when taking the moving image of the subject. The invention of a medical image diagnostic device configured to play back only the portion of the moving image with which the marker with the number larger than the set number is associated while skipping a portion with low importance (portion with which the marker with the number smaller than the set number is associated) if it is configured to play back the portion with high importance to which the larger number is set, for example, when the doctor watches the moving image to diagnose.

A field of the ultrasonic diagnostic device, the magnetic resonance imaging device such as an MRI (magnetic resonance imaging) and the like having a long history from when this is introduced into a facility such as a hospital and the diagnosis by using the devices is started is the field in which a diagnostic routine by the doctor and an imaging routine by an imaging technician are established, so that the imaging technician knows an important portion in the moving image for the doctor to diagnose. Therefore, the imaging technician may determine importance of the portion while watching the moving image being taken at the time of imaging as described above. Since the marker of the large number is adequately associated with the portion of high importance, so that the doctor may play back only the important portion based on the marker as described above.

However, an attempt to take the dynamic image of the site to be examined of a subject by using the semiconductor image sensor such as the FPD to apply the same to the diagnosis, that is to say, technology of dynamic analysis as described above is a new examination method having a short history, so that it cannot be said that the diagnostic routine by the doctor and the imaging routine by the imaging technician are currently established. First of all, it is not currently known which spot of the dynamic image of the site to be examined the doctor focuses on to diagnose.

In such a situation, it is dangerous for the imaging technician to decide the importance by arbitrary determination as in JP 2002-095640 A described above; the imaging technician does not know the spot in the dynamic image to focus on. The doctor does not know the spot in the dynamic image to focus on for utilizing the same in the diagnosis unless actually watching the dynamic image to diagnose.

Summary of the Invention

The present invention has been achieved in view of the above, and an object thereof is to provide a console and a dynamic image taking/diagnostic system capable of automatically finding the important spot in a dynamic image.

To achieve the abovementioned object, according to an aspect, a console reflecting one aspect of the present invention comprises: a display unit which plays back a dynamic image formed of a plurality of frame images to display according to operation of an operator; and a learning unit which, when detecting that operation of possibly expressing interest is performed by the operator during playback of the dynamic image, performs statistical processing on a value of an index regarding a dynamic state of a site to be examined and/or change in time of the value of the index in a frame image displayed on the display unit at that time to learn appearance frequency of the value of the index and/or the pattern of the change in time of the value of the index.

Brief Description of the Drawings

The above and other objects, advantages and features of the present invention will become more fully understood from the detailed description given hereinbelow and the appended drawings which are given by way of illustration only, and thus are not intended as a definition of the limits of the present invention, and wherein:

    Fig. 1 is a view illustrating an entire configuration of a dynamic image taking/diagnostic system according to this embodiment;
    Fig. 2 is a flowchart illustrating an imaging control processing executed by a controller of an imaging console;

Fig. 3 is a flowchart illustrating an image analytical processing executed by a controller of a diagnostic console;

Fig. 4 is a view illustrating frame images of a plurality of time phases taken in one respiratory cycle;

Fig. 5 is a graph indicating change in time of height in a vertical direction of the diaphragm and change in time of an average signal value of a certain small block, the graph illustrating phase delay time;

Fig. 6 is a view illustrating a lung field region and the small block in a reference image;

Fig. 7 is a view illustrating an example of a map of a delay degree relative to the change in time of the height in the vertical direction of the diaphragm and the map illustrating an abnormality determination result;

Fig. 8 is a graph indicating change in time of a cardiac wall position and change in time of an average signal value of a certain small block, the graph illustrating phase delay time;

Fig. 9 is a view illustrating an example of a map of a delay degree relative to the change in time of the cardiac wall position and the map illustrating an abnormality determination result;

Fig. 10A is a view illustrating a configuration of the console;

Fig. 10B is a view illustrating an example of a diagnostic screen displayed on a display means of the console;

Fig. 11 is a view illustrating an example of a histogram prepared for each type of index;

Fig. 12A is a graph indicating an example of an index value with a specific pattern in change in time of the index value; and

Fig. 12B is a view illustrating an example of a virtual voting box prepared for each type of the index.

Description of the Preferred Embodiments

Hereinafter, an embodiment of a console and a dynamic image taking/diagnostic system according to the present invention will be described with reference to the drawings. However, the scope of the invention is not limited to the illustrated examples.

Meanwhile, a case of dynamically imaging a chest of a subject to analyze a pulmonary ventilation function and a pulmonary blood stream by the dynamic image taking/diagnostic system is hereinafter mainly described, but in addition to this, it is also possible to configure to dynamically image an extension/contraction state of a joint region of a human body being the subject to analyze an extension/contraction function, for example; the present invention is not limited to the case of analyzing the pulmonary ventilation function and the pulmonary blood stream.

[Configuration of Dynamic Image Taking/Diagnostic system 100]

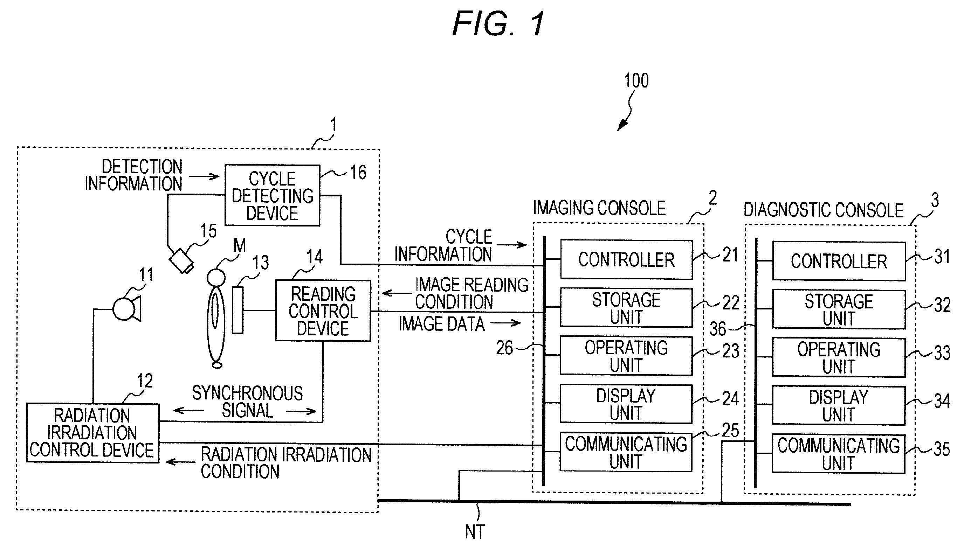

A configuration of the dynamic image taking/diagnostic system according to this embodiment is described. Fig. 1 illustrates an entire configuration of a dynamic image taking/diagnostic system 100 of this embodiment. As illustrated in Fig. 1, the dynamic image taking/diagnostic system 100 is such that an imaging device 1 and an imaging console 2 are connected to each other by means of a communication cable and the like and the imaging console 2 and a diagnostic console 3 are connected to each other through a communication network NT such as a LAN (local area network). Each device forming the dynamic image taking/diagnostic system 100 meets DICOM (digital image and communications in medicine) standards and communication among the devices is performed in conformity with the DICOM.

[Configuration of Imaging Device 1]

The imaging device 1 is a device which images a dynamic state of the chest having periodicity (cycle) such as morphological change such as expansion and contraction of the lung in accordance with respiratory motion and heartbeat, for example. Dynamic imaging is performed by continuously irradiating the chest of the human body with radiation such as an X-ray to obtain a plurality of images (that is to say, by continuously imaging). A series of images obtained by the continuous imaging is referred to as a dynamic image. Each of a plurality of images forming the dynamic image is referred to as a frame image.

The imaging device 1 is provided with a radiation source 11, a radiation irradiation control device 12, a radiation detecting unit 13, a reading control device 14, a cycle detecting sensor 15, a cycle detecting device 16 and the like as illustrated in Fig. 1.

The radiation source 11 irradiates a subject M with the radiation (X-ray) under the control of the radiation irradiation control device 12. The radiation irradiation control device 12 connected to the imaging console 2 controls the radiation source 11 to perform radiation imaging based on a radiation irradiation condition input from the imaging console 2. The radiation irradiation condition input from the imaging console 2 includes a pulse rate, a pulse width, and a pulse interval at the time of continuous irradiation, imaging starting/finishing timing, a value of X-ray tube current, a value of X-ray tube voltage, a filter type and the like, for example. The pulse rate is the number of times of radiation irradiation per second and this conforms to a frame rate to be described later. The pulse width is radiation irradiation time per radiation irradiation. The pulse interval is a time period from a start of one radiation irradiation to a start of next radiation irradiation in the continuous imaging and this conforms to a frame interval to be described later.

The radiation detecting unit 13 is formed of a semiconductor image sensor such as a FPD. The FPD includes a glass substrate and the like, for example, and a plurality of pixels which detects the radiation emitted from the radiation source 11 to be transmitted through at least the subject M according to its intensity and converts the detected radiation to an electric signal to accumulate is arranged in a matrix pattern in a predetermined position on the substrate. Each pixel is formed of a switching unit such as a TFT (thin film transistor), for example.

The reading control device 14 is connected to the imaging console 2. The reading control device 14 controls the switching unit of each pixel of the radiation detecting unit 13 based on an image reading condition input from the imaging console 2 to switch reading of the electric signal accumulated in each pixel and reads the electric signals accumulated in the radiation detecting unit 13 to obtain image data. The image data is the frame image. The reading control device 14 outputs the obtained frame image to the imaging console 2.

The image reading condition includes the frame rate, the frame interval, a pixel size, an image size (matrix size) and the like, for example. The frame rate is the number of frame images obtained per second and this conforms to the pulse rate. The frame interval is a time period from a start of obtaining operation of one frame image to a start of the obtaining operation of a next frame image in the continuous imaging and conforms to the pulse interval.

Herein, the radiation irradiation control device 12 and the reading control device 14 are connected to each other and communicate synchronous signals to each other to synchronize radiation irradiation operation with image reading operation.

The cycle detecting sensor 15 detects a state of the respiratory motion of the subject M and outputs detection information to the cycle detecting device 16. A respiration monitoring belt, a CCD (charge coupled device) camera, an optical camera, a spirometer and the like may be applied as the cycle detecting sensor 15, for example.

The cycle detecting device 16 detects the number of respiratory cycles and a current state in one cycle of the respiratory motion (out of inspiration, a changing point from inspiration to expiration, expiration, a changing point from expiration to inspiration, for example) based on the detection information input by the cycle detecting sensor 15 and outputs a detection result (cycle information) to a controller 21 of the imaging console 2. The cycle detecting device 16 makes timing at which the detection information indicating that a pulmonary status is on the changing point from inspiration to expiration is input by the cycle detecting sensor 15 (respiration monitoring belt, CCD camera, optical camera, spirometer and the like) a base point of one cycle, for example, and recognizes a time period until timing at which this state is next detected as one cycle.

[Configuration of Imaging Console 2]

The imaging console 2 outputs the radiation irradiation condition and the image reading condition to the imaging device 1 to control the radiation imaging by the imaging device 1 and the reading operation of a radiation image and displays the dynamic image obtained by the imaging device 1 for confirming positioning by an imaging technician and confirming whether this is the image suitable for diagnosis.

The imaging console 2 is provided with the controller 21, a storage unit 22, an operating unit 23, a display unit 24, and a communicating unit 25 connected to one another by a bus 26 as illustrated in Fig. 1.

The controller 21 is formed of a CPU (central processing unit), a RAM (random access memory) and the like. The CPU of the controller 21 reads a system program and various processing programs stored in the storage unit 22 to develop in the RAM and executes various pieces of processing such as imaging control processing to be described later according to the developed programs, thereby performing concentrated control of operation of each unit of the imaging console 2 and the radiation irradiation operation and the reading operation of the imaging device 1 in response to operation of the operating unit 23.

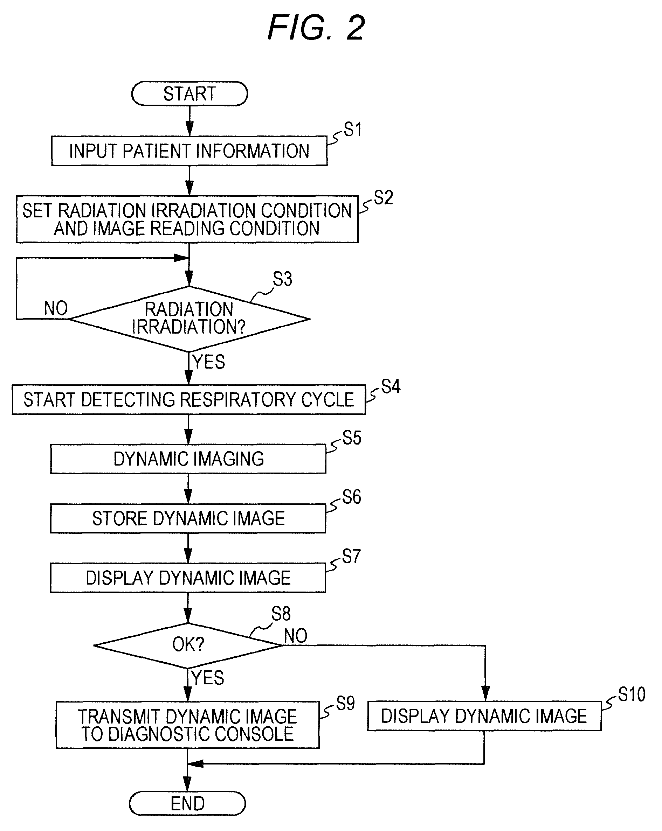

The storage unit 22 is formed of a non-volatile semiconductor memory, a hard disk and the like. The storage unit 22 stores various programs executed by the controller 21, parameters required for executing the processing by the programs, or data such as a processing result. For example, the storage unit 22 stores an imaging control processing program for executing the imaging control processing illustrated in Fig. 2. The storage unit 22 stores the radiation irradiation condition and the image reading condition in association with a site to be examined. The various programs are stored in a mode of a readable program code and the controller 21 sequentially executes operation according to the program code.

The operating unit 23 is provided with a keyboard including a cursor key, a number input key, and various function keys and a pointing device such as a mouse and outputs an instruction signal input by key operation on the keyboard and mouse operation to the controller 21. The operating unit 23 may also be provided with a touch panel on a display screen of the display unit 24; in this case, this outputs the instruction signal input through the touch panel to the controller 21.

The display unit 24 formed of a monitor such as an LCD (liquid crystal display) and a CRT (cathode ray tube) plays back to display the dynamic image and displays various data and an instruction content and the like input from the operating unit 23 in response to an instruction of a display signal input from the controller 21.

The communicating unit 25 provided with a LAN adapter, a modem, a TA (terminal adapter) and the like controls transmission/reception of data to/from each device connected to the communication network NT.

[Configuration of Diagnostic Console 3]

The diagnostic console 3 is a moving image processing device for obtaining the dynamic image from the imaging console 2 and displaying the obtained dynamic image such that a doctor reads the same to diagnose. Meanwhile, there also is a case in which the dynamic image is temporarily transmitted from the imaging console 2 to an external system such as a PACS (picture archiving and communication system) and thereafter the diagnostic console 3 obtains the dynamic image from the external system. There also is a case in which the imaging console 2 and the diagnostic console 3 are integrated, that is to say, one device serves as the imaging console 2 and the diagnostic console 3.

The diagnostic console 3 is provided with a controller 31, a storage unit 32, an operating unit 33, a display unit 34, and a communicating unit 35 connected to one another through a bus 36 as illustrated in Fig. 1.

The controller 31 is formed of a CPU, a RAM and the like. The CPU of the controller 31 reads a system program and various processing programs stored in the storage unit 32 to develop in the RAM and executes various pieces of processing such as image analytical processing to be described later according to the developed programs, thereby performing concentrated control of operation of each unit of the diagnostic console 3 in response to operation of the operating unit 33. The controller 31 realizes an image analyzing means by executing the image analytical processing to be described later.

The storage unit 32 is formed of a non-volatile semiconductor memory, a hard disk and the like. The storage unit 32 stores various programs such as an image analytical processing program for executing the image analytical processing by the controller 31, parameters required for executing the processing by the programs, or data such as a processing result. The various programs are stored in a mode of a readable program code and the controller 31 sequentially executes the operation according to the program code.

The operating unit 33 is provided with a keyboard including a cursor key, a number input key, and various function keys and a pointing device such as a mouse and outputs an instruction signal input by key operation on the keyboard and mouse operation to the controller 31. The operating unit 33 may also be provided with a touch panel on a display screen of the display unit 34; in this case, this outputs the instruction signal input through the touch panel to the controller 31.

The display unit 34 formed of a monitor such as an LCD and a CRT plays back to display the dynamic image and displays various data and an instruction content and the like input from the operating unit 33 in response to an instruction of a display signal input from the controller 31.

The communicating unit 35 provided with a LAN adaptor, a modem, a TA and the like controls the transmission/reception of the data to/from each device connected to the communication network NT.

[Operation of Dynamic Image Taking/Diagnostic System 100]

Operation in the above-described dynamic image taking/diagnostic system 100 is next described.

[Operation of Imaging Device 1 and Imaging Console 2]

Imaging operation by the imaging device 1 and the imaging console 2 is first described. Fig. 2 illustrates the imaging control processing executed by the controller 21 of the imaging console 2. The imaging control processing is executed by cooperation of the controller 21 and the imaging control processing program stored in the storage unit 22.

The operating unit 23 of the imaging console 2 is operated by the imaging technician and patient information (name, body height, body weight, age, sex and the like of the patient) of an imaging target (subject M) is input (step S1).

Next, the radiation irradiation condition is read from the storage unit 22 to be set in the radiation irradiation control device 12 and the image reading condition is read from the storage unit 22 to be set in the reading control device 14 (step S2). The frame rate (pulse rate) of five frames/second or higher is preferable. In general, delay time difference of ventilation in the lung field and the pulmonary blood stream is one second or shorter, so that five frames/second or higher rate is required for decomposing phase delay time into a plurality of stages (at least five or more stages) to represent with a high degree of accuracy. Meanwhile, the phase delay time to be described later is disclosed in detail in JP 2010-268979 A and the like described above, for example, so that please refer to the same for more detail.

Next, an instruction of the radiation irradiation by the operation of the operating unit 23 is waited, and when the radiation irradiation instruction is input by the operating unit 23 (step S3: YES), an instruction to start detecting the cycle is output to the cycle detecting device 16 and the cycle detecting sensor 15 and the cycle detecting device 16 starts detecting the cycle of the respiratory motion of the subject M (step S4).

When a predetermined state (for example, the changing point from inspiration to expiration) is detected by the cycle detecting device 16, an instruction to start imaging is output to the radiation irradiation control device 12 and the reading control device 14 and the dynamic imaging is started (step S5). That is to say, the radiation source 11 emits the radiation at the pulse interval set in the radiation irradiation control device 12 and the frame image is obtained by the radiation detecting unit 13. When a predetermined number of dynamic cycles are detected by the cycle detecting device 16, the controller 21 outputs an instruction to finish imaging to the radiation irradiation control device 12 and the reading control device 14 and the imaging operation is stopped.

The frame images obtained by imaging are sequentially input to the imaging console 2 to be stored in the storage unit 22 in association with the numbers indicating the order of imaging (step S6) and displayed on the display unit 24 (step S7). The imaging technician confirms the positioning and the like by the displayed dynamic image to determine whether the image suitable for the diagnosis is obtained by imaging (imaging OK) or retake is required (imaging NG). Then, the imaging technician operates the operating unit 23 to input a determination result.

When the determination result indicating "imaging OK" is input by predetermined operation of the operating unit 23 (step S8; YES), information such as an identification ID for identifying the dynamic image, the patient information, the site to be examined, the radiation irradiation condition, the image reading condition, the number indicating the order of imaging, and the cycle information is added to each of a series of frame images obtained by the dynamic imaging (for example, written in a header of the image data in a DICOM format) to be transmitted to the diagnostic console 3 through the communicating unit 25 (step S9). Then, this procedure is finished. On the other hand, when the determination result indicating "imaging NG" is input by predetermined operation of the operating unit 23 (step S8; NO), a series of frame images stored in the storage unit 22 is deleted (step S10) and this procedure is finished.

[Operation of Diagnostic Console 3]

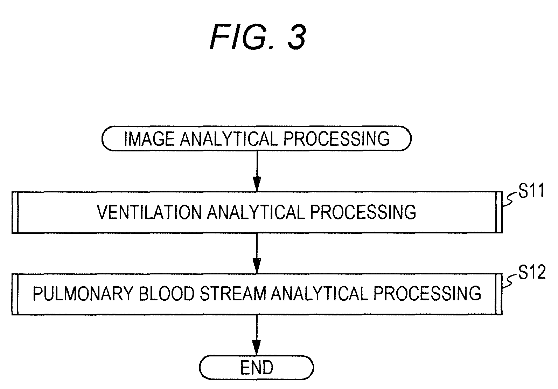

Next, operation in the diagnostic console 3 is described. In the diagnostic console 3, when a series of frame images of the dynamic image is received from the imaging console 2 through the communicating unit 35, the image analytical processing illustrated in Fig. 3 is executed by cooperation of the controller 31 and the image analytical processing program stored in the storage unit 32.

Meanwhile, it is not required to configure such that both of ventilation analytical processing and pulmonary blood stream analytical processing are necessarily performed in the diagnostic console 3; it is also possible to configure to perform any one of them or extension/contraction function analytical processing of the joint region of the human body described above, for example. The ventilation analytical processing and the pulmonary blood stream analytical processing in the image analytical processing are disclosed in detail in JP 2010-268979 A described above, so that please refer to the same for further detail.

Hereinafter, a case in which the ventilation analytical processing (step S11) and the pulmonary blood stream analytical processing (step S12) are performed in the image analytical processing as illustrated in Fig. 3 is simply described. In the image analytical processing, values of various indices and the like are calculated.

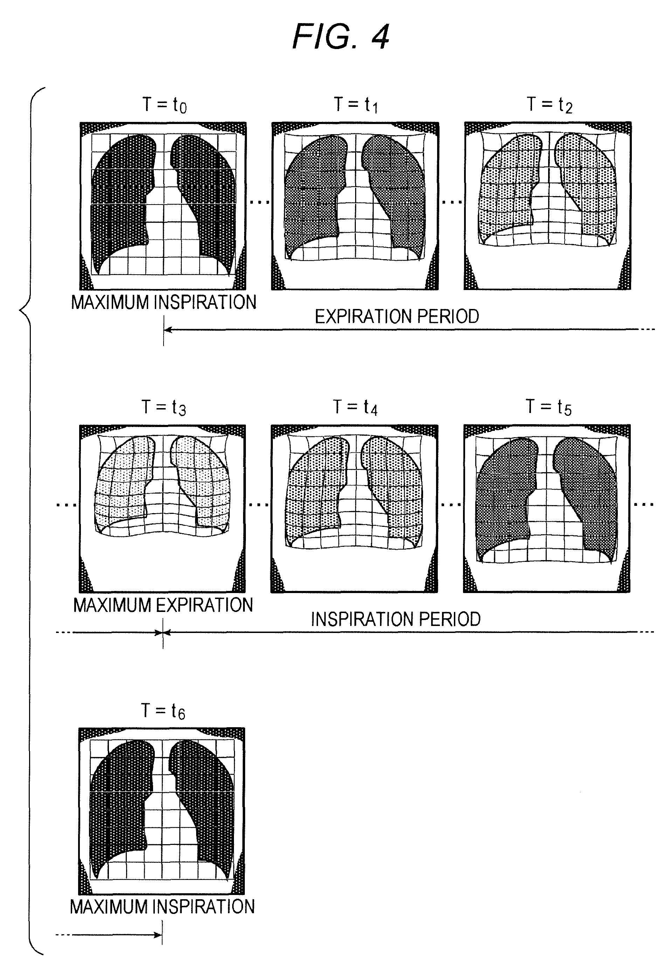

For example, in the ventilation analytical processing (step S11), height in a vertical direction of the diaphragm is calculated as the value of the index from the chest dynamic image obtained by imaging the lung field. The diaphragm promotes the respiratory motion of the lung by vertical motion thereof. For example, as illustrated in the frame images of a plurality of time phases $T$ ($T = t_0$ to $t_6$) taken in one respiratory cycle illustrated in Fig. 4, the respiratory cycle is formed of an expiration period ($T = t_0$ to $t_3$) in which a position of the diaphragm is raised and an inspiration period ($T = t_3$ to $t_6$) in which the position of the diaphragm is lowered. In this manner, in the chest dynamic image, the motion in the vertical direction of the diaphragm serves as the index indicating the respiratory motion of the lung and the height in the vertical direction of the diaphragm serves as the value of the index indicating the respiratory motion of the lung (hereinafter, referred to as an index value).

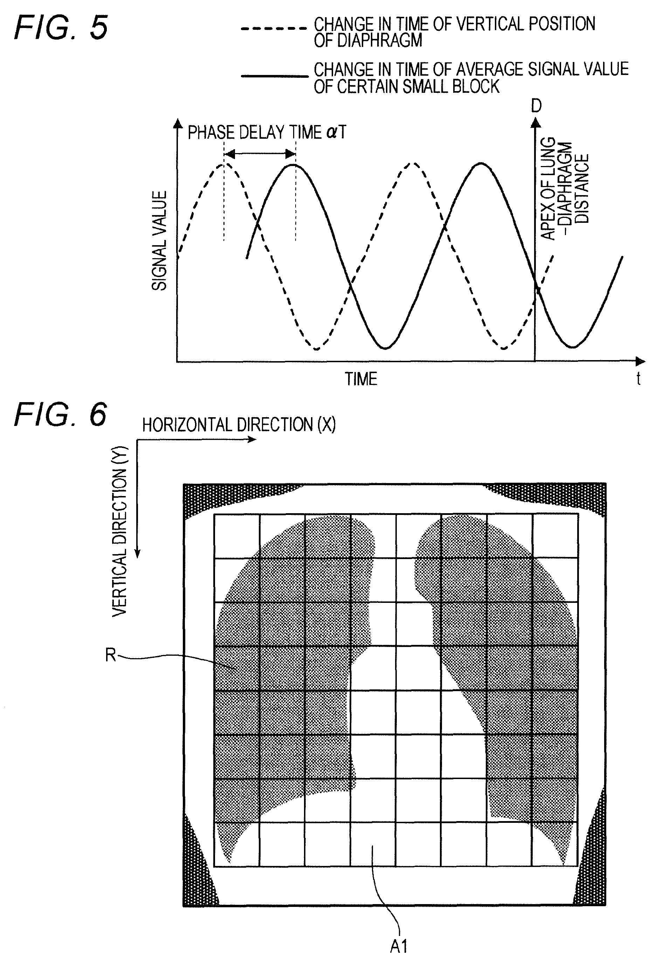

As is clear from Fig. 4, a vertical position of the apex of lung is substantially the same in the expiration period and the inspiration period in each frame image. Therefore, the height in the vertical direction of the diaphragm as the index value may be represented as a distance $D$ in the vertical direction between the apex of lung and the diaphragm. As indicated by a broken line in Fig. 5, for example, it is possible to obtain change in time of the height $D$ in the vertical direction of the diaphragm (apex of lung - diaphragm distance $D$) by plotting temporal transition of the distance $D$. Meanwhile, in Fig. 5, elapsed time $t$ from the start of the dynamic imaging and the distance $D$ are represented along the abscissa and the ordinate, respectively. Hereinafter, the distance $D$ is sometimes referred to as height $D$ in the vertical direction of the diaphragm.

It is also possible to calculate various values based on the height $D$ in the vertical direction of the diaphragm as the index value indicating the respiratory motion.

For example, when air is taken in the lung by respiration, a signal value of each pixel of the lung field becomes larger, and when air is discharged from the lung, the signal value of each pixel of the lung field becomes smaller. The signal value increases or decreases by the respiration with slight delay from the height $D$ in the vertical direction of the diaphragm (apex of lung - diaphragm distance $D$) as the index value as indicated by a solid line in Fig. 5. By calculating this temporal delay as phase delay time $\alpha T$ by using a time delay time calculating method by Fourier series expansion, for example, and focusing on the phase delay time $\alpha T$, it is understood whether there is abnormality in the ventilation function in a lung field region.

That is to say, a lung field region R expanding and contracting according to the respiration in each frame image as illustrated in Fig. 4 is shifted such that each position of the lung field region R is in each corresponding position of the lung field region R of a reference image illustrated in Fig. 6 (determined to be the frame image taken the first and the like, for example), thereby forming the frame image in which a size and a position of the lung field region R are the same across respective frame images.

By dividing the lung field region R of each frame image into a plurality of regions (small blocks A1), calculating an average signal value (density average value) of the pixels in each small block A1, and plotting temporal transition of the average signal value, change in time of the average signal value of each small block A1 is obtained as indicated by the solid line in Fig. 5. By analyzing the temporal delay of a profile of the change in time of the average signal value of each small block A1 from a profile of the change in time of the height D in the vertical direction of the diaphragm indicated by the broken line in Fig. 5, it is possible to calculate the phase delay time $\alpha T$ for each small block A1.

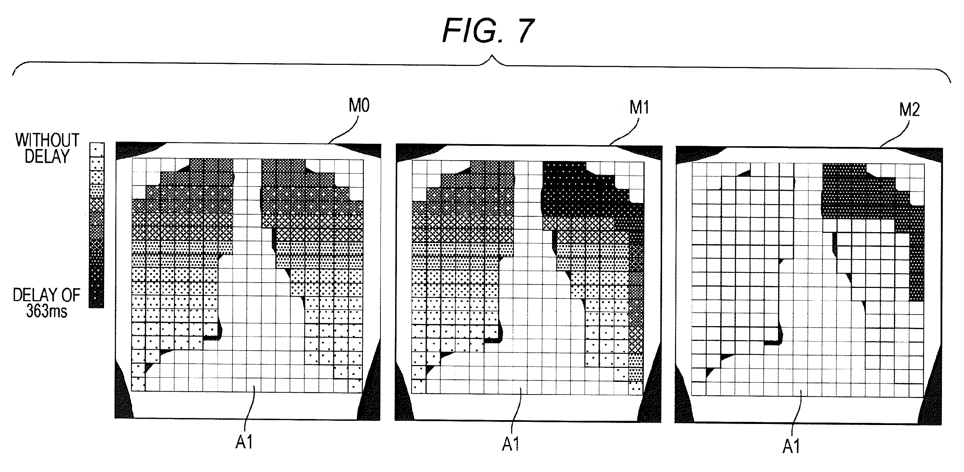

The phase delay time $\alpha T$ is substantially constant according to the distance from the diaphragm when the pulmonary ventilation function is normal, but if there is a spot where the ventilation function is abnormal in the lung field region, the phase delay time $\alpha T$ becomes longer in this portion. Therefore, as illustrated in Fig. 7, for example, abnormality determination to determine whether the phase delay time $\alpha T$ of each small block A1 calculated in the above-described manner (meanwhile, the phase delay time $\alpha T$ of each small block A1 is indicated by magnitude of a luminance value to be illustrated as a map M1 in Fig. 7) becomes longer than a threshold according to the distance from the diaphragm.

When there is the small block A1 in which the phase delay time $\alpha T$ is longer than the threshold in the abnormality determination as indicated in a deep color in a map M2 illustrating an abnormality determination result in Fig. 7 (actually, this is displayed in red and the like), it is understood that this portion is the spot where the ventilation function is locally deteriorated in the lung field of the subject M. In this manner, the map M2 and the like may be used in diagnosing a ventilation defect of the lung such as chronic obstructive pulmonary disease (COPD), interstitial pneumonia, pneumothorax and the like in a department of respiratory disease.

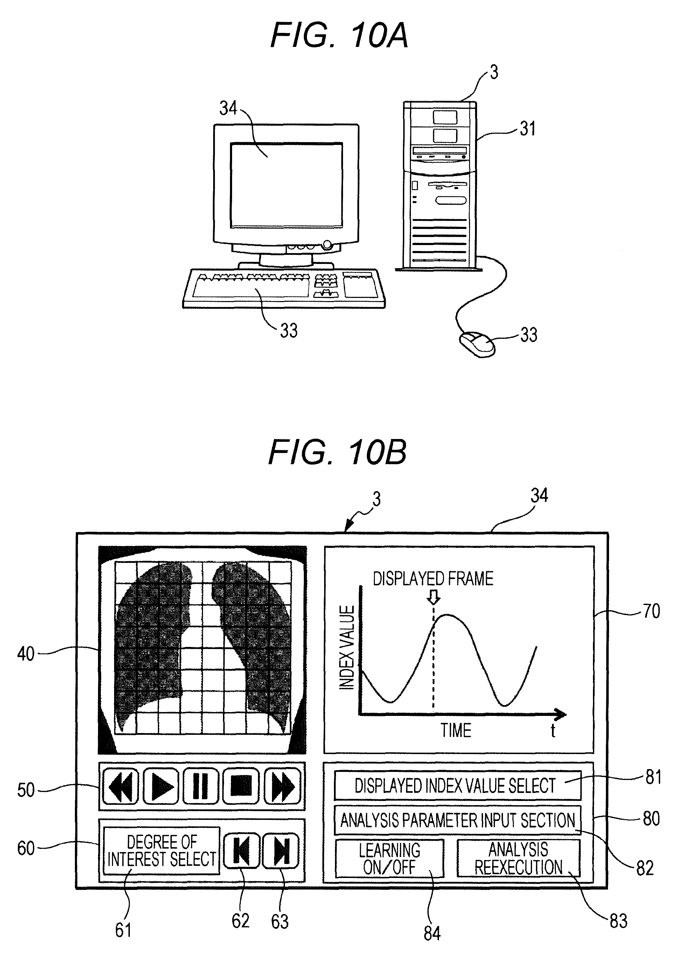

When displaying the above-described maps M1 and M2 on an index value display section 70 on a diagnostic screen to be described later (refer to Fig. 10B to be described later), if a standard delay degree map M0 illustrating standard phase delay time $\alpha T$ of each small block A1 in the normal lung field in which the ventilation function is not deteriorated is displayed together with the maps M1 and M2 as illustrated in Fig. 7, the doctor and the like who watches the same may easily and adequately identify the spot where the ventilation function is locally deteriorated in the lung field of the subject M.

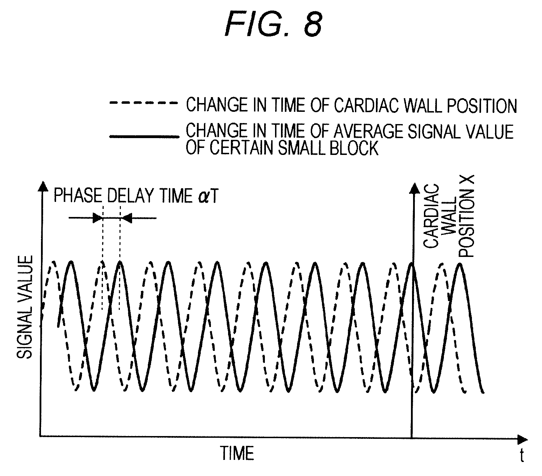

On the other hand, in the pulmonary blood stream analytical processing (step S12), cardiac wall motion serves as the index indicating the heartbeat and a cardiac wall position is calculated as the index value indicating the heartbeat from the chest dynamic image obtained by imaging the lung field as described above. That is to say, although not illustrated, it is possible to find a cardiac region from each frame image to specify a reference position of the cardiac wall of the left heart ventricle and calculate a cardiac wall position X and change in time thereof based on a position thereof in a horizontal direction (X coordinate: refer to Fig. 6), for example, as indicated by a broken line in Fig. 8, for example.

It is also possible to calculate various values based on the cardiac wall position X as the index value indicating the heartbeat.

For example, the signal value of the lung field in each frame image changes depending on the pulmonary blood stream amount generated by the heartbeat and the pulmonary blood stream amount changes according to the cardiac wall position X as the index value indicating the heartbeat described above. The signal value increases or decreases by the pulmonary blood stream amount with slight delay from the cardiac wall position X as the index value as indicated by a solid line in Fig. 8. By calculating the temporal delay as the phase delay time $\alpha T$ by using the time delay time calculating method by Fourier series expansion, for example, and focusing on the phase delay time $\alpha T$, it is understood whether there is abnormality in the pulmonary blood stream.

Therefore, by dividing the lung field region R of each frame image into a plurality of small blocks A1 (refer to Fig. 6), calculating the average signal value (density average value) of the pixels in each small block A1, and plotting the temporal transition of the average signal value, the change in time of the average signal value of each small block A1 is obtained as indicated by the solid line in Fig. 8. By analyzing the temporal delay of the profile of the change in time of the average signal value of each small block A1 from a profile of the change in time of the cardiac wall position X indicated by the broken line in Fig. 8, it is possible to calculate the phase delay time $\alpha T$ for each small block A1.

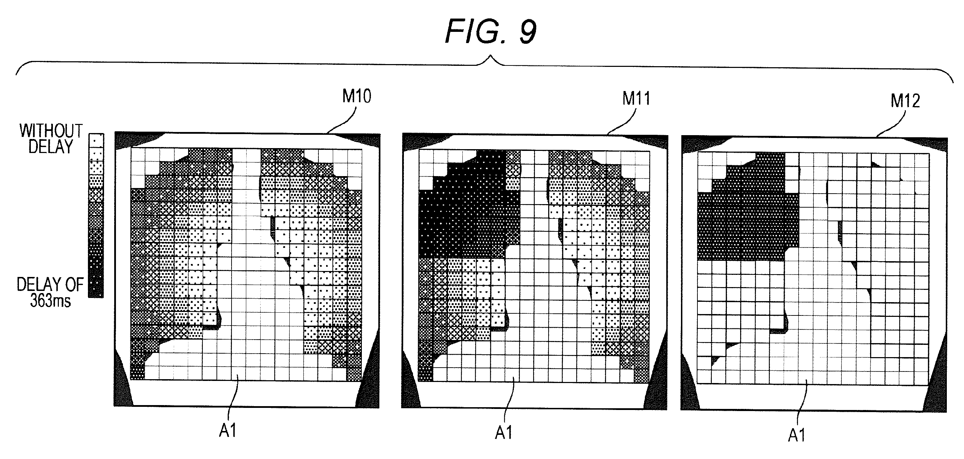

In this case, the phase delay time $\alpha T$ is substantially constant according to a distance from a central portion of the heart as illustrated in a standard delay degree map M10 in Fig. 9, but if there is a spot where the pulmonary blood stream function is abnormal in the lung field region, the phase delay time $\alpha T$ becomes longer in this position. Therefore, as illustrated in Fig. 9, for example, abnormality determination to determine whether the phase delay time $\alpha T$ of each small block A1 calculated in the above-described manner (meanwhile, the phase delay time $\alpha T$ of each small block A1 is indicated by magnitude of a luminance value to be illustrated as a map M11 in Fig. 9) becomes longer than a threshold according to the distance from the central portion of the heart.

When there is the small block A1 in which the phase delay time $\alpha T$ becomes longer than the threshold in the abnormality determination as indicated in a deep color in a map M12 illustrating an abnormality determination result in Fig. 9 (actually, this is displayed in red and the like), it is understood that this portion is the spot where the pulmonary blood stream is locally abnormal in the lung field of the subject M. In this manner, the map M12 and the like may be used in diagnosing the pulmonary blood stream in acute pulmonary thromboembolism (deep-vein thrombosis) and the like in a cardiovascular department, for example.

Meanwhile, in this case also, when displaying the above-described maps M11, M12 and the like on the index value display section 70 on the diagnostic screen to be described later (refer to Fig. 10B described later), if the standard delay degree map M10 is displayed together with the maps M11 and M12 as illustrated in Fig. 9, the doctor and the like who watches the same may easily and adequately identify the spot where the pulmonary blood stream is locally abnormal in the lung field of the subject M.

As described above, in the ventilation analytical processing (step S11) and the pulmonary blood stream analytical processing (step S12) in the image analytical processing in the diagnostic console 3, the height D in the vertical direction of the diaphragm (and the change in time thereof) as the value of the index indicating the respiration motion of the lung and the cardiac wall position X (and the change in time thereof) as the value of the index indicating the heartbeat are calculated. The maps M1 and M2, the maps M11 and M12 and the like are generated based on the values of the indices.

When performing the image analytical processing on the dynamic image obtained by imaging the extension/contraction state of the joint region of the human body, for example, it is possible to configure to perform the image analysis by making extension/contraction motion of the joint region the index and making an angle of the joint region the value of the index, for example. A result of the image analysis may be used in treating and diagnosing the joint region in departments of surgery and orthopedic surgery, for example.

[Regarding Learning]

A configuration of the console according to this embodiment is next described. An action of the console according to this embodiment is also described. Meanwhile, although a case in which the console according to this embodiment is the above-described diagnostic console 3 is hereinafter described and this is simply referred to as the console 3, the console according to this embodiment may also include the imaging console 2 described above as described later.

As described above, different from fields of an ultrasonic diagnostic device and a magnetic resonance imaging device disclosed in JP 2002-095640 A, technology of dynamic analysis described above is a new examination method having a short history, so that it cannot be said that a diagnostic routine by the doctor and an imaging routine by the imaging technician are currently established. Currently, it is not known which spot in the dynamic image of the site to be examined the doctor focuses on to diagnose.

Therefore, in this embodiment, the console 3 learns which feature the frame image in which the doctor expresses interest out of the dynamic image has, that is to say, learns in which value of which index displayed in the frame image the doctor expresses interest.

The type and the value of the index in which the doctor expresses interest are considered to be important elements for the diagnosis. Therefore, learning by the console 3 in the above-described manner may eventually lead to obtaining the index and its value serving as a basis for finding an important spot in the dynamic image by the console 3.

Specifically, in this embodiment, the console 3 includes a display means and a learning means. In the above-described example (refer to Fig. 1), the display unit 34 of the diagnostic console 3 corresponds to the display means and the controller 31 (that is to say, the CPU) corresponds to the learning means. Therefore, they are hereinafter referred to as the display means 34 and the learning means 31. In this embodiment, the console 3 includes a selecting unit such as a keyboard and a mouse as illustrated in Fig. 10A. They are hereinafter referred to as a selecting means 33 in consideration of the fact that the keyboard and the mouse are referred to as the operating unit 33 in the above-described example.

In this embodiment, the console 3 plays back the dynamic image to display on the display means 34 according to the operation of the operator (doctor, in this case). The learning means 31 of the console 3 is configured to learn appearance frequency of the index value by performing statistical processing on the value of the index (hereinafter, referred to as the index value) regarding the dynamic state of the site to be examined in the frame image displayed on the display means 34 at that time when detecting that operation of possibly expressing interest is performed by the operator during the playback of the dynamic image.

Meanwhile, the operation of possibly expressing interest is intended to mean the operation performed by the operator when the operator expresses interest; however, actually, it is not always true that the operator performs the operation because the operator expresses interest, so that this is referred to as the "operation of possibly expressing interest". However, it is hereinafter principally described as "operation of expressing interest" for simplifying the description.

In the above-described case, the site to be examined may be not only the lung but also the joint region and the like of the human body, for example, and the index value regarding the dynamic state thereof may be not only the height D in the vertical direction of the diaphragm and the cardiac wall position X when the site to be examined is the lung but also the angle of the joint region and the like when the site to be examined is the joint region of the human body. Hereinafter, a specific example is described.

[Example of Diagnostic Screen]

The console 3 (in this case, the diagnostic console 3) is configured to display the diagnostic screen illustrated in Fig. 10B, for example, on the display means 34. In this example, a dynamic image display section 40 on which each frame image of the dynamic image is displayed is provided on an upper left portion of the diagnostic screen. Meanwhile, in Fig. 10B, a case in which the dynamic image in a state in which the lung field region R is divided into the small blocks A1 as illustrated in Figs . 4 and 6 is displayed on the dynamic image display section 40 is illustrated, it is also possible to display the dynamic image itself and the dynamic image may be displayed in various states on the dynamic image display section 40.

A dynamic image playback operating section 50 is provided below the dynamic image display section 40 on the diagnostic screen, and it is possible to rewind, play back, pause, stop, fast-forward the dynamic image displayed on the dynamic image display section 40 by clicking a button icon displayed thereon. Although not illustrated in Fig. 10B, it is also possible to configure such that the number of the frame image may be input, for example, and to configure to play back from the frame image of the number on the dynamic image display section 40 when the number of the frame image is input. Meanwhile, a frame of interest skip operating section 60 provided below the dynamic image playback operating section 50 on the diagnostic screen is described later.

The index value display section 70 is provided on an upper right portion of the diagnostic screen. In the index value display section 70, a graph in which the index value indicated by the broken line in Figs. 5 and 8 (that is to say, the distance D between the apex of lung and the diaphragm (that is to say, the height D in the vertical direction of the diaphragm) in Fig. 5, and the cardiac wall position X in Fig. 8) is represented along the ordinate and time t is represented along the abscissa is displayed, for example.

In the index value display section 70, characters of "displayed frame", an arrow, a line and the like in the graphical display indicate the frame to which the frame image of the dynamic image displayed on the dynamic image display section 40 belongs such that the operator (doctor) may understood at a glance to which frame the frame image of the dynamic image currently displayed on the dynamic image display section 40 belongs or what value the index value at that time is.

Meanwhile, it is also possible to configure to indicate to which frame the frame image of the dynamic image displayed on the dynamic image display section 40 belongs by the number of the frame image, by a progress bar or the like, for example, in place of indicating the same by the characters, the arrow, the line and the like on the index value display section 70.

An analytical operating section 80 is provided below the index value display section 70 on the diagnostic screen. Although not illustrated, when clicking a displayed index value selecting button 81 of the analytical operating section 80, for example, a pop-up window is displayed on the diagnostic screen such that the index value to be displayed on the index value display section 70 (for example, the height D in the vertical direction of the diaphragm and the cardiac wall position X described above) may be selected on the window.

Similarly, when clicking an analysis parameter button 82 of the analytical operating section 80, a pop-up window is displayed on the diagnostic screen such that the parameter used in the above-described dynamic analysis may be changed to be input on the window. When clicking an analysis re-executing button 83 after inputting the analysis parameter, the dynamic analysis is executed again based on the analysis parameter input in the above-described manner and the dynamic image as a result of the reexecution is displayed on the dynamic image display section 40. Meanwhile, although it is not described above, the parameters used in various types of arithmetic operation, cutoff frequencies of a low pass filter and a high pass filter, a pixel pitch, a size of the small block A1 and the like, for example, may be used as the analysis parameter; it is also possible to change a range and the like in which the dynamic analysis is performed.

It is also possible to configure such that the parameter regarding the display of the dynamic image such as brightness of an entire dynamic image and a scale factor when displaying the dynamic image on the dynamic image display section 40 is changed by the analysis parameter input section 82, and it is also possible to configure to provide another button for this.

In this embodiment, when the operator (doctor) clicks a learning on/off button 84 of the analytical operating section 80 on the diagnostic screen, it is put into a "learning on" state and the learning means 31 of the console 3 starts learning processing. Meanwhile, when the learning on/off button 84 is clicked again, it is put into a "learning off" state and the learning means 31 stops the learning processing.

Meanwhile, it is also possible to configure such that various data such as a longitudinal/lateral size of the lung field and a cardiothoracic ratio in the frame image displayed on the dynamic image display section 40, for example, may be measured by operation on the diagnostic screen, for example.

[Regarding Detection of Operation of Expressing Interest]

As described above, in this embodiment, when the learning means 31 of the console 3 detects that the operation of expressing interest (operation of possibly expressing interest) is performed by the operator (doctor, in this case) during the playback of the dynamic image, this performs the statistical processing on the index value regarding the dynamic state of the site to be examined in the frame image displayed on the display means 34 (that is to say, the dynamic image display section 40 on the diagnostic screen) at that time to learn the appearance frequency of the index value.

At that time, when there is the frame image which the doctor who is the operator wants to watch in further detail in the dynamic image, the doctor who is the operator sometimes performs operation to variously change a method of analyzing the frame image by executing the analysis again on the frame image while changing the parameter used in the dynamic analysis, that is to say, the parameters used in the various types of arithmetic operation, the cutoff frequencies of the low pass filter and the high pass filter, the pixel pitch, the size of the small block A1, and the range in which the dynamic analysis is performed described above.

Therefore, the learning means 31 may be configured to detect that the operator (doctor) performs the operation of expressing interest by the fact that the operator changes the parameter used in the dynamic analysis to input, for example.

That is to say, in the example of the diagnostic screen described above, when the learning means 31 detects that the operator clicks the analysis parameter input section 82 of the analytical operating section 80 to display the pop-up window in a state in which a certain frame image in the dynamic image is displayed on the dynamic image display section 40, thereby performing operation to change the parameter used in the dynamic analysis on the dynamic image to input, it is detected that the operator (doctor) performs the operation of expressing interest.

When the operator finds the frame image of interest in the dynamic image, the operator often pauses the frame image to watch carefully or rewinds to watch the frame image when passing through the same. Although not illustrated in the dynamic image playback operating section 50 on the above-described diagnostic screen (refer to Fig. 10B), when it is configured such that the dynamic image may be played back in slow motion, for example, the operator sometimes plays back the frame image of interest in the dynamic image in slow motion to watch carefully.

Therefore, the learning means 31 may also be configured to detect that the operation of expressing interest is performed by the operator by the fact that the operation to pause, rewind, and play back in slow motion is performed by the operator during the playback of the dynamic image, for example.

That is to say, in the example of the diagnostic screen described above, the learning means 31 detects that the operator performs the operation to pause or rewind the dynamic image by clicking the button icon to pause or rewind of the dynamic image playback operating section 50 during the playback of the dynamic image, and according to this, it is detected that the operation of expressing interest is performed by the operator.

Furthermore, the operator often changes the parameter regarding the display of the dynamic image such as the brightness of the entire dynamic image and the scale factor when the dynamic image is displayed on the dynamic image display section 40 when the operator wants to watch the frame image in detail. Therefore, the learning means 31 may also be configured to detect that the operator performs the operation of expressing interest by the fact that the operator changes the parameter regarding the display of the dynamic image to input, for example.

For example, when the operator outputs information of the frame image displayed on the dynamic image display section 40 on the diagnostic screen to the external system such as an electronic medical chart, a reading report, or the PACS, it is understood that the operator is interested in the frame image. Therefore, it is also possible to configure to detect that the operation of expressing interest is performed by the operator also when the operator performs such a process.

Alternatively, also when the operator inputs the information in the electronic medical chart and the like on the console 3 in the state in which the frame image is displayed on the dynamic image display section 40 on the diagnostic screen or when the operator performs operation to request to retake through the console 3 in this state, it is understood that the operator is interested in the frame image. Therefore, it is also possible to configure to detect that the operation of expressing interest is performed by the operator also when the operator performs such process or operation.

In this manner, in this embodiment, the learning means 31 is configured to detect that the operation of expressing interest is performed by the operator when the operation which the operator might perform on the console 3 when the operator is interested in the frame image of interest in the dynamic image is performed. By configuring in this manner, it becomes possible to adequately detect that the operator performs the operation of expressing interest.

Meanwhile, for example, when the operator clicks "pause" for taking a bathroom break or rewinds the dynamic image for watching the same again from the first, the operator is not interested in the frame image at which the operator pauses or from which the operator rewinds. Therefore, it may be configured not to detect that the operation of expressing interest is performed by the operator when the pause continues for a long time such as for one minute or longer, when the number of frames rewound is larger than a predetermined number, or when the playback in slow motion is performed for a long time.

When the parameter used in the dynamic analysis is changed in the above-described manner also, there is a case in which it is less likely that the operator is interested in the frame image displayed at that time if operation to simply return the parameter to is default setting (initial setting) is performed, for example. Therefore, it is also possible to configure not to detect that the operator performs the operation of expressing interest when the parameter is simply returned to its default setting.

Furthermore, it is also possible to configure not to detect that the operation of expressing interest is performed by the operator when the operation to pause, rewind, or play back in slow motion is performed but to detect this when one more operation is performed in addition to this. That is to say, it is also possible to configure to detect this when the operation to pause is performed after rewinding, or when the parameter used in the dynamic analysis or the parameter regarding the display of the dynamic image is changed to be input after the pause. It goes without saying that modification for more surely detecting that the operation of expressing interest is performed by the operator is appropriately made in this manner.

[Regarding Statistical Processing on Index Value]

In this embodiment, when the learning means 31 of the console 3 detects that the operator performs the operation of expressing interest as in the above-described manner, this performs the statistical processing on the index value regarding the dynamic state of the site to be examined in the frame image displayed on the display means 34 at that time to learn the appearance frequency of the value of the index and/or a pattern of the change in time of the value of the index.

Specifically, in this embodiment, as illustrated in Fig. 10B, when the dynamic image is played back on the diagnostic screen, the index value such as the distance D between the apex of lung and the diaphragm (refer to Fig. 5), the cardiac wall position X (refer to Fig. 8), or the angle of the joint region selected by the operator through the click of the displayed index value selecting button 81 is displayed on the index value display section 70 on the diagnostic screen. Meanwhile, selecting the index value by the selecting means 33 such as the mouse and the keyboard by the operator is equivalent to selecting the type of the index.

The learning means 31 may be configured to learn the value of the index regarding the dynamic state of the site to be examined of the type selected by the selecting means 33, that is to say, the index value selected through the click of the displayed index value selecting button 81 to be displayed on the index value display section 70 in the example of the diagnostic screen described above when this detects that the operation of expressing interest is performed by the operator who watches the frame image displayed on the display means 34.

In this embodiment, the statistical processing is performed in the following manner. Meanwhile, although a case in which a histogram and a virtual voting box provided on a memory are used is hereinafter described as the statistical processing, any statistical processing may be performed as long as the appearance frequency of the index value may be figured out.

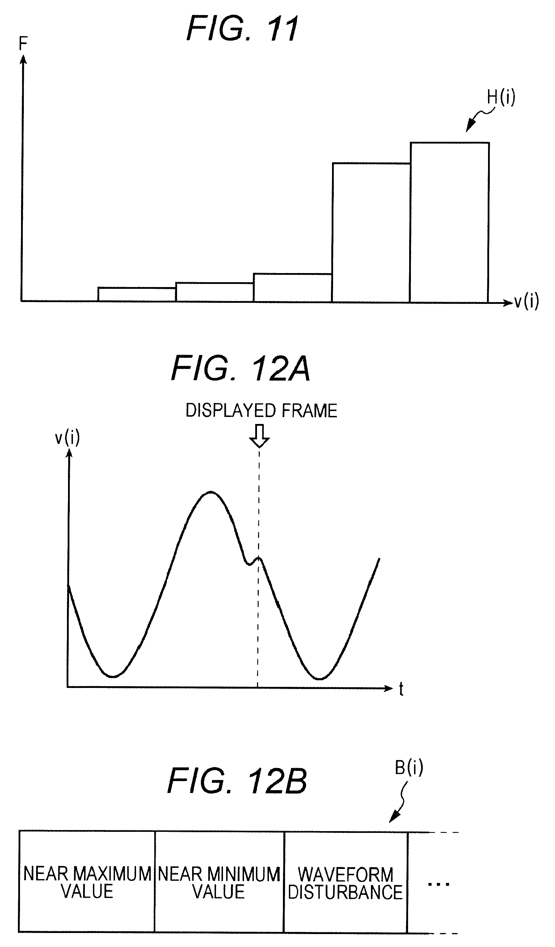

If the type of the index is represented by i and the index value is represented by v(i), for example, as illustrated in Fig. 11, a histogram H(i) in which the index values v(i) are classified with a predetermined class width is prepared in advance in the memory such as the RAM of the console 3 for each type of the index i. In this embodiment, the learning means 31 votes the index value v(i) of the index of the selected type i to a corresponding class of a corresponding histogram H(i) when detecting that the operation of expressing interest is performed by the operator in the above-described manner during the playback of the dynamic image.

In this embodiment, the learning means 31 of the console 3 learns which value (index value v(i)) of which index (type of the index i) appearing in the frame image the doctor is interested in by learning the appearance frequency of the index value v(i) by voting the index value v(i) regarding the dynamic state of the site to be examined appearing in the frame image displayed on the display means 34 when the operator performs the operation of expressing interest to the histogram by performing the statistical processing in this manner.

Meanwhile, the class width of the histogram H(i) is appropriately determined according to the type of the index i and the like: the histogram is divided into large, middle, and small three classes or into more fine classes.

Since the dynamic analysis is the new examination method having the short history as described above, it is supposed that an inexperienced doctor performs various operations in a trial-and-error manner, and a learning result might be of a low utility value if there remains the learning result based on such operation. Therefore, it is also possible to configure to delete the index value v(i) with predetermined time after the voting out of the index values v(i) voted to the histogram H(i) therefrom, for example.

In the above-described example, it is supposed that the operator often watches the frame image with interest in the index value v(i) selected by clicking the displayed index value selecting button 81 on the diagnostic screen (that is to say, the index value v(i) displayed on the index value display section 70), so that a case in which the learning means 31 is configured to learn the value of the index of the selected type i (that is to say, the index value v(i)) when detecting that the operator performs the operation of expressing interest as described above is described.

However, as described above, even when the operator focuses on a certain frame image of the dynamic image, the operator is not necessarily interested in the index value v(i) displayed on the index value display section 70 on the diagnostic screen in the frame image, and it is also possible that the operator watches the frame image with interest in another index value v(i) (specifically, the dynamic state with which the other index value v(i) associates) without switching the display of the index value v(i) of the index value display section 70 to the other index value v(i) (that is to say, without switching the type of index i to another type i).

Therefore, it is also possible to configure such that the learning means 31 simultaneously learns in parallel all the index values v(i) specified in advance (that is to say, all the index values v(i) which might be selected when the displayed index value selecting button 81 is clicked, for example) in the above-described manner without limiting the index value v(i) to be learned to the index value v(i) selected by the operator (that is to say, the index value v(i) displayed on the index value display section 70) as described above.

There might be a case in which the operator (doctor) does not express interest in a certain index value v(i) itself but in a specific pattern of the change in time dv(i)/dt of the index value v(i) (for example, refer to a portion indicated as "displayed frame" in Fig. 12A) as illustrated in Fig. 12A, for example, while watching the frame image of the dynamic image.

Therefore, in this case, a virtual voting box B (i) in which patterns are classified for voting the pattern of the change in time dv(i)/dt of the index value v(i) as illustrated in Fig. 12B is prepared for each index value v(i) in advance in the memory such as the RAM of the console 3, for example, in place of or in parallel with configuring to vote the index value v(i) to the histogram H (i) in the above-described manner.

The learning means 31 learns the appearance frequency of the pattern of the change in time dv(i)/dt of the index value v(i) by analyzing the pattern of the change in time dv(i)/dt of the index value v(i) to vote to a corresponding box of a corresponding virtual voting box B (i) when detecting that the operation of expressing interest is performed by the operator during the playback of the dynamic image. By configuring in this manner, the learning means 31 of the console 3 learns which pattern of the change in time dv(i)/dt of the index value v(i) appearing in the frame image the doctor is interested in. At that time, it is appropriately determined according to the type of the index i and the like how to classify the patterns to each box of the virtual voting box B(i).

It is considered that much information useful for the diagnosis is included in the index value v(i) and the pattern of the change in time dv(i)/dt of the index value v(i) appearing in the frame image in which the operator (the doctor in this case) who watches the dynamic image expresses interest. Therefore, it is considered that a range (class) of the index values v(i) and the pattern of the change in time dv(i)/dt of the index value v (i) with large numbers of votes to the histogram H(i) and the virtual voting box B(i) and with high appearance frequencies lead to the information useful for the diagnosis.

Therefore, by configuring in the above-described manner, it becomes possible that the learning means 31 automatically and adequately finds the important spot (that is to say, the frame image) in the dynamic image including much information useful for the diagnosis from a voting result to each class of the histogram H (i) and to each box of the virtual voting box B(i) (that is to say, the appearance frequency) by performing the statistical processing on the index value v(i) and the pattern of the change in time dv (i) /dt of the index value v (i) in the frame image in which the operator expresses interest and learning the appearance frequency thereof.

[Effect]

As described above, according to the console 3 according to this embodiment, it is configured such that, when the learning means 31 detects that the operation of expressing interest (operation of possibly expressing interest) is performed by the operator during the playback of the dynamic image, this performs the statistical processing on the index value v(i) of the index regarding the dynamic state of the site to be examined and the pattern of the change in time dv(i)/dt of the index value v(i) in the frame image displayed on the display means 34 at that time, thereby learning the appearance frequency of the index value v(i) and the pattern of the change in time dv(i)/dt of the index value v(i).

Therefore, the learning means 31 may learn the appearance frequency by performing the statistical processing on the index value v(i) and the pattern of the change in time dv(i)/dt of the index value v(i) in the frame image in which the operator expresses interest, thereby automatically and adequately finding the important spot (that is to say, the frame image) in the dynamic image including much information useful for the diagnosis by using the learning result (that is to say, the appearance frequency in each class of the histogram H(i) and each box of the virtual box B(i) in the above-described example).

Therefore, when diagnosing by using the new examination method of the dynamic analysis with the short history, it becomes possible to adequately find the spot (frame image) in the dynamic image including much information useful for the diagnosis in which the doctor who diagnoses is interested as the important spot in the dynamic image, different from JP 2002-095640 A described above in which the imaging technician decides the same while determining importance, so that the learning result is useful for the diagnosis.

[Regarding Type of Index]

Meanwhile, although the height D in the vertical direction of the diaphragm, the cardiac wall position X, and the angle of the joint region are described as the types of the index in the above-described embodiment, the type is not limited thereto.

For example, when the dynamic analysis of the chest dynamic image is performed, the change in time of the average signal value of the pixel (refer to the solid line in Fig. 5), the change in time of the signal value by the pulmonary blood stream amount (refer to the solid line in Fig. 8) and the like are obtained for each small block A1 obtained by dividing the lung field region R as described above, it is also possible to configure to learn the average signal value of the pixel and the signal value by the pulmonary blood stream amount for each small block A1 as the index and the index value v (i) and to learn the pattern of the change in time dv(i)/dt of the index value v (i) .

As described above, in the diagnostic console 3 of the dynamic image taking/diagnostic system 100 according to this embodiment, the map M1 illustrating the phase delay time αT regarding the pulmonary ventilation function in each small block A1, the map M2 illustrating the abnormality determination result (refer to Fig. 7), the map M11 illustrating the phase delay time αT regarding the pulmonary blood stream amount of each small block A1, and the map M12 illustrating the abnormality determination result (refer to Fig. 9) are formed.

When the doctor of the department of respiratory disease watches the dynamic image, the doctor might see the ventilation function in the portion in which the pulmonary ventilation function is abnormal (that is to say, the change in time of the average signal value of the pixel in the small block A1 in the abnormal portion) or see the difference from the ventilation function of the normal portion. When the doctor of the cardiovascular department watches the dynamic image, the doctor might see the change in time of the average signal value of the pixel in the small block A1 in the portion in which the pulmonary blood stream amount is abnormal or see the difference in the pulmonary blood stream amount from the normal portion.

Therefore, for example, it is also possible to configure to make the average signal value of the pixel in the small block A1 in the portion in which the pulmonary ventilation function and the pulmonary blood stream amount are abnormal the index and the index value v(i) specific to the portion or to make the difference in the average signal value of the pixel between the small block A1 of the abnormal portion and the small block A1 of the normal portion the index value and the index value v(i), thereby making the index, the index value v(i), or the pattern of the change in time dv(i)/dt of the index value v(i) obtained by using the result of the abnormality determination and the like the learning target.

Furthermore, in the embodiment illustrated in Fig. 1, the cycle detecting device 16 detects the number of respiratory cycles and the current state in one cycle of the respiratory motion (out of inspiration, the changing point from inspiration to expiration, expiration, and the changing point from expiration to inspiration, for example) based on the detection information input by the cycle detecting sensor 15. Therefore, it is also possible to configure to utilize the information as the index and the index value v(i) and make the index value v(i) and the pattern of the change in time dv(i)/dt of the index value v(i) the learning target.

In this case, the doctor does not watch the image detected by the cycle detecting sensor 15 while the dynamic image is taken at the time of imaging; the doctor watches the dynamic image played back on the display means 34 of the console 3 and performs the operation of expressing interest (operation of possibly expressing interest) when there is the frame image of interest after the dynamic image is taken.

Therefore, when it is configured in the above-described manner, the image and the like detected by the cycle detecting sensor 15 at the time of imaging is stored, for example, and the console 3 associates each frame image of the dynamic image with each image detected by the cycle detecting sensor 15 at the same time (or at substantially the same time) .

Then, the console 3 may be configured to find the image detected by the cycle detecting sensor 15 corresponding to the frame image displayed at that time when the doctor who watches the dynamic image performs the operation of expressing interest to learn the index value v(i) and the pattern of the change in time dv(i)/dt of the index value v(i) in the image.

Since the dynamic analysis is the new examination method having the short history and it is not established which index value v(i) is suitable for diagnosing which disease, so that it is desirable to configure such that many indices which might contribute to the diagnosis may be learned at least when it is learned in the above-described manner for diagnosing.

[Regarding Setting of Operation of Expressing Interest]

In the above-described embodiment, it is described on the assumption that the operation to change the parameter used in the dynamic analysis and the operation to pause, rewind, and play back in slow motion are set in advance in the console 3, for example, as the operation by which the operator (doctor and the like) expresses interest (possibly expresses interest) and the console 3 is configured to detect that the operation of expressing interest is performed by the operator when any set operation is performed.

However, the operation is not required to be set in advance, or it is also possible to configure to input new type of operation to set in the console 3 as the operation of expressing interest by the operator after starting learning in addition to the operation set in advance.

When configuring in this manner, even when the operation which is not supposed before starting learning is performed as the operation of expressing interest by the operator, such operation may be adequately added to be set as the operation of expressing interest by the operator, and it becomes possible to more adequately detect the operation of expressing interest by the operator (operation of possibly expressing interest) to adequately learn.

[Regarding Utilization of Learning Result]

It is possible to configure to utilize the learning result by the console 3 in the above-described manner as follows, for example.

[Application 1: Cue When Playing Back According to Degree of Interest]

For example, it is possible to use the voting result to the histogram H(i) (refer to Fig. 11) and the voting box B(i) (refer to Fig. 12B), that is to say, a degree F in each class of the histogram H(i) and the number of votes to each box of the voting box B(i) (that is to say, the appearance frequency) as the marker (bookmark) disclosed in JP 2002-095640 A. Specifically, it may be configured in the following manner, for example.

The console 3 displays a degree of interest selecting button 61 on the frame of interest skip operating section 60 provided below the dynamic image playback operating section 50 on the diagnostic screen as an input means capable of inputting a degree of interest as illustrated in Fig. 10B. When the operator clicks the degree of interest selecting button 61, a pop-up window is displayed on the diagnostic screen and a degree of interest I may be input on the window. The degree of interest I is input as a value within a range from one to ten, for example.

In this case, the larger the number, the higher the degree of interest. The learning means 31 of the console 3 calculates a ratio $\gamma$ [%] from the top according to following equation (1) when the degree of interest I is input.

$$\gamma = 100 - (I - 1) \times 10 \ \ldots \ (1)$$

The learning means 31 specifies the class and the box to which the index value v(i) and the pattern of the change in time dv(i)/dt of the index value v(i) included in the calculated ratio $\gamma$ from the top belong, the value and the pattern out of the index values v(i) and the patterns of the change in time dv(i)/dt of the index value v(i) voted to the histogram H(i) (refer to Fig. 11) and the box of the virtual voting box B(i) (refer to Fig. 12B) corresponding to the type i of the index value v(i) selected through the click of the displayed index value selecting button 81 of the analytical operating section 80 as described above (that is to say, the index value v(i) graphically displayed in the index value display section 70 in the above-described case).

It is possible to configure to start playing back the dynamic image from the frame image in which the index value v(i) and the pattern of the change in time dv(i)/dt of the index value v(i) belonging to the class of the histogram H(i) or the box of the virtual voting box B(i) with a high degree of interest I specified in the above-described manner appear for the first time (that is to say, cue) when playing back the dynamic image.

By configuring in this manner, it becomes possible to cue to play back the dynamic image from the frame image in which the index value v(i) and the pattern of the change in time dv(i)/dt of the index value v(i) according to the degree of interest of the operator appear. Therefore, it becomes possible that the operator plays back to watch the dynamic image from the frame image which the operator wants to watch while saving an effort of searching the frame image in which the index value v(i) and the pattern of the change in time dv(i)/dt of the index value v (i) according to the degree of interest appears by playing back the dynamic image from the first.

Meanwhile, in this embodiment, when buttons 62 and 63 indicating playback skip operation displayed on the frame of interest skip operating section 60 on the diagnostic screen are clicked, it is possible to skip a next frame image to cue or to skip to a previous frame image to cue out of the frame images corresponding to the index value v(i) and the degree of interest I input by the operator.

[Application Example 2: Regarding Application of Learning Result by Diagnostic Console in Imaging Console]

It is also possible to configure to transmit the learning result by the learning means 31 of the diagnostic console 3 performed in the above-described manner (that is to say, the histogram H(i) and the virtual voting box B(i) and the appearance frequency in each class and each box) to the imaging console 2 (refer to Fig. 1) and apply the learning result in the imaging console 2.

As described above, in the imaging console 2, the imaging technician confirms the dynamic image displayed on the display unit 24 to determine whether the image suitable for the diagnosis is obtained (imaging OK) or the retake is required (imaging NG) when the dynamic image is taken.

Therefore, it is possible to configure such that the controller 21 of the imaging console 2 plays back the dynamic image in slow motion in a portion of the frame image in which a class value and the pattern with a large number of votes of the index values v(i) and the patterns of the change in time dv(i)/dt of the index value v(i) appears such that the imaging technician may easily confirm and plays back the dynamic image at fast speed in a portion of other frame images, for example, based on the learning result transmitted from the diagnostic console 3 when the imaging technician confirms the dynamic image.

By configuring in this manner, the portion of the frame image in which much information useful for the diagnosis is included of the dynamic image is played back in slow motion, so that the imaging technician may surely assess the portion of such frame image to adequately determine whether the image suitable for the diagnosis is obtained. Since the dynamic image is played back at fast speed in the portion of the other frame image, so that it becomes possible to rapidly perform confirming operation while adequately determining whether the site to be imaged is surely taken in the frame image.

In this manner, by configuring to transmit the learning result by the learning means 31 of the diagnostic console 3 performed in the above-described manner to the imaging console 2 (refer to Fig. 1) and apply the learning result in the imaging console 2, it becomes possible to adequately take the dynamic image and perform the confirming operation by the imaging technician and adequately take the dynamic image suitable for the diagnosis.

[Regarding Learning by Imaging Console 2 Itself]

Meanwhile, although a case in which the learning result obtained by the diagnostic console 3 is transmitted to the imaging console 2 to be applied in the imaging console 2 is described in the above-described application example 2, it is also possible to configure to learn by the imaging console 2 itself regardless of the diagnostic console 3.

When configuring to learn by the imaging console 2, it is possible to configure to learn as in the above-described case of learning by the diagnostic console 3. In this case, the above-described console 3 is replaced with the console 2 (imaging console 2) and the controller 21 of the imaging console 2 serves as the learning means and the display unit 24 serves as the display means.

The imaging technician who is the operator might pause, rewind, or play back in slow motion the dynamic image when determining whether the image suitable for the diagnosis is obtained in the confirming operation of the dynamic image. Therefore, when the operator performs such operation, it is understood that the operator is interested in this frame image. Therefore, it is also possible to configure to detect that the operation of expressing interest is performed by the operator when the operator performs such operation.

It is also understood that the frame image displayed on the display means 24 of the console 2 when the imaging technician who is the operator determines that the dynamic image is not suitable for the diagnosis and the retake is required is also the frame image in which the operator is interested. Therefore, it is also possible to configure to detect that the operation of expressing interest is performed by the operator also when the operator performs input operation of an instruction to retake.

This is the processing performed when the dynamic image is taken and the dynamic analysis by the diagnostic console 3 is not yet performed on the dynamic image. Therefore, the learning means 21 (controller 21) of the imaging console 2 performs calculation processing of the index value v(i) and the like before performing the statistical processing.

That is to say, the learning means 21 performs the image processing as in the case of the diagnostic console 3 described above or performs simpler image processing on the taken dynamic image, specifies the lung field region R in the frame image, and calculates the average signal value of the pixel and contrast in each small block obtained by dividing the lung field region R in small blocks (they are not necessarily the same as the above-described small blocks A1) or a region of interest ROI set in the lung field region R in addition to the height D in the vertical direction of the diaphragm and the cardiac wall position X, thereby automatically calculating the index value v(i) (that is to say, without the operation by the imaging technician).

Then, by configuring to automatically learn the index values v(i) simultaneously in parallel and perform the statistical processing thereon, it becomes possible to automatically and adequately learn the appearance frequency of the value v(i) of the index and the pattern of the change in time dv(i)/dt of the index value v(i) in which the imaging technician is interested when determining whether the image suitable for the diagnosis is obtained in the dynamic image.

It becomes possible to play back the dynamic image in slow motion in the portion of the frame image in which the imaging technician is interested and to play back the dynamic image at fast speed in a portion other than this when the imaging technician confirms the dynamic image, for example, based on the learning result, and it becomes possible to obtain a useful effect such that the imaging technician may adequately and rapidly perform the confirming operation of the dynamic image.

[Another Configuration Example]

Meanwhile, the learning results obtained by the consoles 2 and 3 according to this embodiment might be utilized for establishing the diagnostic routine (diagnostic console 3) by the doctor and the imaging routine (imaging console 2) by the imaging technician in the future, so that it is possible to configure to provide the histogram H(i) and the like for each disease name or each treatment department to learn for each disease name or each treatment department.

Other than this, it is also possible to configure to learn for each doctor, each patient, each dosing data, each symptom, or each site to be examined, for example.

Although not illustrated, it is also possible to configure to display the appearance frequency and ranking of each class of the histogram H(i) and each box of the voting box B(i) of the displayed index value v(i) or to display portions of the respective classes in different colors on the index value display section 70 and the like on the diagnostic screen described above (refer to Fig. 10B).

Furthermore, as described above, it is also possible to configure to indicate the frame to which the frame image of the dynamic image displayed on the dynamic image display section 40 belongs by the progress bar, for example; at that time, as for the index value v(i) displayed on the index value display section 70 on the diagnostic screen, it is possible to display corresponding portions of the progress bar in different colors corresponding to the appearance frequency and the ranking according to the appearance frequency and the ranking of the index value v (i) in each frame image of the dynamic image.

In a state in which a certain index value v(i) is displayed on the index value display section 70 on the diagnostic screen as described above, when the operator clicks the analysis parameter button 82 of the analytical operating section 80 on the diagnostic screen to input the analysis parameter such as the parameters used in various types of arithmetic operation and the cutoff frequencies of the low pass filter and the high pass filter to execute the dynamic analysis again, the index value v(i) is voted to the corresponding class of the histogram H(i).

It is possible to configure such that, when the console 3 continuously plays back the dynamic image in this state, if the index value v(i) becomes the index value belonging to the above-described class, the console 3 applies the above-described input parameter to the frame image to display.

Meanwhile, it goes without saying that the present invention is not limited to the above-described embodiment and the like and may be appropriately changed without departing from the gist of the present invention.

Although the present invention has been described and illustrated in detail, it is clearly understood that the same is by way of illustrated and example only and is not to be taken by way of limitation, the scope of the present invention being interpreted by terms of the appended claims.

**Claims**

1.    A console (3) **characterized by** comprising:

> a display means (24, 34) which plays back a dynamic image formed of a plurality of frame images to display according to operation of an operator; and
> a learning means (21, 31) which, when detecting that operation of possibly expressing interest is performed by the operator during playback of the dynamic image, performs statistical processing on a value of an index regarding a dynamic state of a site to be examined and/or change in time of the value of the index in a frame image displayed on the display means (24, 34) at that time to learn appearance frequency of the value of the index and/or the pattern of the change in time of the value of the index.

2.    The console (3) according to claim 1, comprising:

> a selecting means (33) capable of selecting a type of the index, **characterized in that** the learning means (21, 31) performs the statistical processing on the value of the index and/or the pattern of the change in time of the value of the index of the type selected when the learning means (21, 31) detects that the operation of possibly expressing interest is performed by the operator during the playback of the dynamic image.

3.    The console (3) according to claim 1 or 2, **characterized in that**

> the learning means (21, 31) includes a histogram (H(i)) classified with a predetermined class width and/or a virtual voting box (B(i)) in which patterns are classified for voting the pattern of the change in time of the value of the index for each type of the index and votes the value of the index and/or the pattern of the change in time of the value of the index to a corresponding class of the histogram (H(i)) and/or a corresponding box (B(i)) of the virtual voting box to perform the statistical processing when detecting that the operation of possibly expressing interest is performed by the operator during the playback of the dynamic image.

4.    The console (3) according to any one of claims 1 to 3, **characterized in that** the operation of possibly expressing interest is set in advance and/or is input to be set.

5.    The console (3) according to any one of claims 1 to 4, **characterized in that** the operation of possibly expressing interest is that a parameter regarding display of the dynamic image is changed to be input by the operator or that operation to pause, rewind, or play back in slow motion is performed during the playback of the dynamic image.

6.    The console (3) according to any one of claims 1 to 4, **characterized in that** the operation of possibly expressing the interest is that a parameter used in dynamic analysis is changed to be input by the operator or information of the frame image displayed on the display means (24, 34) is output.

7.    The console (3) according to any one of claims 1 to 4, **characterized in that** the operation of possibly expressing interest is that the operator inputs an instruction to retake.

8.    The console (3) according to claim 3, comprising:

> a selecting means (33) capable of selecting the type of the index; and
> an input means capable of inputting a degree of interest, **characterized in that**
> the learning means (21, 31) specifies the class of the histogram (H(i)) and/or the box of the virtual voting box (B(i)) to which the value of the index and/or the pattern of the change in time of the value of the index included in a ratio from the top corresponding to the input degree of interest belongs, the value and the pattern out of the values of the index and/or the patterns of the change in time of the value of the index voted to the histogram (H(i)) and/or the box of the virtual voting box (B(i)) corresponding to the selected type of the index, and plays back the dynamic image from the frame image in which the value of the index and/or the pattern of the change in time of the value of the index belonging to the specified class of the histogram (H(i)) and/or the specified box of the virtual voting box (B(i)) appears for the first time.

9. A dynamic image taking/diagnostic system (100) comprising:

the console (3) according to any one of claims 1 to 7 as a diagnostic console (3) used by a doctor for diagnosis; and
an imaging console (2) used by a person who takes an image when taking a dynamic image, **characterized in that**
the console (3) as the diagnostic console (3) transmits a learning result by the learning means (21, 31) to the imaging console (2).

FIG. 1

# FIG. 2

START

INPUT PATIENT INFORMATION ⟋S1

SET RADIATION IRRADIATION CONDITION ⟋S2
AND IMAGE READING CONDITION

NO ◇ RADIATION IRRADIATION? ⟍S3

YES

START DETECTING RESPIRATORY CYCLE ⟋S4

DYNAMIC IMAGING ⟋S5

STORE DYNAMIC IMAGE ⟋S6

DISPLAY DYNAMIC IMAGE ⟋S7

◇ OK? ⟍S8   NO

YES

TRANSMIT DYNAMIC IMAGE ⟋S9     DISPLAY DYNAMIC IMAGE ⟋S10
TO DIAGNOSTIC CONSOLE

END

## FIG. 3

```
         ┌─────────────────────────────────────┐
         │    IMAGE ANALYTICAL PROCESSING       │
         └─────────────────────────────────────┘
                          │
                          ▼
    ┌───────────────────────────────────────────────────┐  S11
    │       VENTILATION ANALYTICAL PROCESSING            │
    └───────────────────────────────────────────────────┘
                          │
                          ▼
    ┌───────────────────────────────────────────────────┐  S12
    │  PULMONARY BLOOD STREAM ANALYTICAL PROCESSING      │
    └───────────────────────────────────────────────────┘
                          │
                          ▼
              ┌─────────────────────┐
              │         END         │
              └─────────────────────┘
```

# FIG. 4

T = t₀       T = t₁       T = t₂

MAXIMUM INSPIRATION

EXPIRATION PERIOD

T = t₃       T = t₄       T = t₅

MAXIMUM EXPIRATION

INSPIRATION PERIOD

T = t₆

MAXIMUM INSPIRATION

FIG. 5

- - - - - CHANGE IN TIME OF VERTICAL POSITION
OF DIAPHRAGM

——— CHANGE IN TIME OF AVERAGE SIGNAL VALUE
OF CERTAIN SMALL BLOCK

PHASE DELAY TIME $\alpha$T

SIGNAL VALUE

D

APEX OF LUNG
-DIAPHRAGM
DISTANCE

TIME

t

FIG. 6

HORIZONTAL DIRECTION (X)

VERTICAL DIRECTION (Y)

R

A1

FIG. 7

WITHOUT
DELAY

DELAY OF
363ms

M0

M1

M2

A1

A1

A1

EP 3 121 747 A1

# FIG. 8

- - - - - - CHANGE IN TIME OF CARDIAC WALL POSITION
———————— CHANGE IN TIME OF AVERAGE SIGNAL VALUE OF CERTAIN SMALL BLOCK

PHASE DELAY TIME $\alpha$T

SIGNAL VALUE

CARDIAC WALL POSITION X

TIME

t

# FIG. 9

WITHOUT
DELAY

DELAY OF
363ms

M10

M11

M12

A1

A1

A1

# FIG. 10A

# FIG. 10B

DISPLAYED FRAME

INDEX VALUE

TIME    t

DISPLAYED INDEX VALUE SELECT — 81

ANALYSIS PARAMETER INPUT SECTION — 80

LEARNING ON/OFF

ANALYSIS REEXECUTION

DEGREE OF INTEREST SELECT

## FIG. 11

H(i)

F

v(i)

## FIG. 12A

DISPLAYED FRAME

v(i)

t

## FIG. 12B

B(i)

| NEAR MAXIMUM VALUE | NEAR MINIMUM VALUE | WAVEFORM DISTURBANCE | ... |
|---|---|---|---|

# EP 3 121 747 A1

**EUROPEAN SEARCH REPORT**

Application Number

EP 16 17 5643

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Peter Phillips ET AL: "Method for Tracking Eye Gaze during Interpretation of Endoluminal 3D CT Colonography: Technical Description and Proposed Metrics for Analysis ¦ Radiology", , 30 March 2013 (2013-03-30), XP055318351, Retrieved from the Internet: URL:http://pubs.rsna.org/doi/full/10.1148/radiol.12120062 [retrieved on 2016-11-10] * page 2 * ----- | 1-9 | INV. G06F19/00 G06K9/00 G06T7/00 |
| A | US 8 843 951 B1 (SHERRETS DOUG [US] ET AL) 23 September 2014 (2014-09-23) * abstract; col. 3 and 6-8 * ----- | 1-9 | |

TECHNICAL FIELDS
SEARCHED      (IPC)

G06F
G06K
G06T

The present search report has been drawn up for all claims

| Place of search | Date of completion of the search | Examiner |
|---|---|---|
| Munich | 15 November 2016 | Bankwitz, Robert |

## ANNEX TO THE EUROPEAN SEARCH REPORT
## ON EUROPEAN PATENT APPLICATION NO.

EP 16 17 5643

This annex lists the patent family members relating to the patent documents cited in the above-mentioned European search report.
The members are as contained in the European Patent Office EDP file on
The European Patent Office is in no way liable for these particulars which are merely given for the purpose of information.

15-11-2016

| Patent document cited in search report | | Publication date | Patent family member(s) | | Publication date |
|---|---|---|---|---|---|
| US 8843951 | B1 | 23-09-2014 | US | 8843951 B1 | 23-09-2014 |
| | | | US | 9215502 B1 | 15-12-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010268979 A

- JP 2002095640 A